# EUROPEAN PATENT APPLICATION

(11) **EP 3 904 524 A1**
(43) Date of publication of application: **03.11.2021**
(21) Application number: 21170559.5
(22) Date of filing: 26.04.2021
(51) Int. Cl.: C12P 13/00, C12P 7/04, C12N 1/16, C12R 1/645

(54) **METHOD FOR BIOSYNTHESIZING MONOETHANOLAMINE**

(30) Priority: 24.04.2020 CN 202010331691
(71) Applicant: Xiamen Oamic Biotechnology Co., Ltd., Xiamen, Fujian 361026 (CN)
(72) Inventor: ZHOU, Liqin, Xiamen 361026 (CN); FANG, Peihua, Xiamen 361026 (CN); WANG, Shuning, Xiamen 361026 (CN); YANG, Meixue, Xiamen 361026 (CN); XING, Chenguang, Xiamen 361026 (CN); LIU, Gang, Xiamen 361026 (CN); ZHAO, Xijing, Xiamen 361026 (CN)
(74) Representative: Verscht, Thomas Kurt Albert

(57) **Abstract**

The present disclosure discloses a method for biosynthesizing monoethanolamine, which comprises the following steps: (1) transferring activated *Torulaspora delbrueckii* OMK-71 into a fermentation medium containing a carbon source used to be metabolized by yeasts to promote growth and/or to produce monoethanolamine and culturing to obtain a fermentation broth; (2) adding the fermentation broth to a reaction buffer containing serine to perform a reaction to obtain a reaction solution; and (3) purifying of the reaction solution obtained in step (2) to obtain monoethanolamine. Torulaspora delbrueckii OMK-71 of the present disclosure can be used to biosynthesize monoethanolamine in large scales, and a monoethanolamine yield can reach 79 g/L or higher.

## Description

### TECHNICAL FIELD OF THE DISCLOSURE

The present disclosure relates to fermentation engineering, and in particular, relates to a method for biosynthesizing monoethanolamine.

### BACKGROUND OF THE DISCLOSURE

Monoethanolamine, also known as 2-aminoethanol, 2-hydroxyethylamine, monoethanolamine, ETA or MEA, is a colorless, viscous liquid with the smell of ammonia. Its derivatives widely exist in nature. For example, phosphatidylethanolamine is a component of biological membranes, and in particular is an important component of prokaryotic membranes. Phosphatidylethanolamine plays an important functional role in blood coagulation. Another example is palmitoylethanolamide, which is a signal molecule that can affect the CB1 receptor in the brain. The molecular formula of monoethanolamine is HOCH₂CH₂NH₂, which comprises a primary amine and a primary alcohol, such that monoethanolamine is hygroscopic and can be miscible with water, most alcohols and polyol. Monoethanolamine is weakly alkaline and can react with acids to form esters or salts.

Monoethanolamine is widely used in industries due to its various characteristics. Monoethanolamine is an indispensable raw material for the productions of detergents, emulsifiers, polishing agents, drugs, corrosion inhibitors and chemical intermediates, and the details are as follows:

Monoethanolamine can be used as a surfactant in personal care products, cosmetics, floor and tile cleaners, laundry detergents, etc. In these products, monethanolamine removes dirt and oil from the skin by dissolving oil and mixing other important ingredients. Since monoethanolamine does not emit a strong pungent odor, monoethanolamine is also a common ingredient in products, such as hair dyes, etc.

Due to its emulsifying properties, monoethanolamine can also be used in industrial applications, such as gas treatment and chemical manufacturing. In the gas treatment process of oil refineries and natural gas, monoethanolamine helps to remove pollutants from gasoline, such as carbon dioxide and hydrogen sulfide. Monoethanolamine can very effectively scrub carbon dioxide emitted from burning coal, methane and biogas. The scrubbing using monoethanolamine reduces carbon emissions and enable traditional coal and biogas industries to be more modern, healthier and more marketable. In the ammonia synthesis process of chemical plants, monoethanolamine is also used to remove carbon dioxide from ammonia. In addition, monoethanolamine reacts with ammonia to obtain ethylenediamine, which is a precursor of the commonly used chelating agent EDTA.

Monoethanolamine is also commonly used in the alkalization of water in the steam cycles of power plants, including nuclear power plants with pressurized water reactors, and is a key component of All Volatile Treatment (AVT) of water. Since monoethanolamine does not accumulate in steam generators (boiler) and cracks due to its volatility, it is relatively evenly distributed throughout whole processes of the steam cycle, water alkalization resulting from monoethanolamine can effectively reduce corrosion of metal components. Monoethanolamine can also adjust pH of the personal care products and the cosmetics to prevent decomposition during storage processes and increase their service life.

In pharmaceutical preparations, monoethanolamine is mainly used to prepare emulsions and provide buffering effects. Monoethanolamine is also an injectable sclerosing agent that can be used as treatment option for hemorrhoids. Two to five milliliters of monoethanolamine oleate can be injected into the mucosae right above the hemorrhoids to enable ulcers to be fixed to mucosae, so that descending of the hemorrhoids from the anal canal is avoided. Monoethanolamine can also be used as plasticizers for plastics, which can increase the toughness of plastics and enable them to become softer at the same time.

Globally, monoethanolamine production is mainly centralized in North America, Europe, China and Asia (excluding China). In 2016, North America produced 647,000 tons of monoethanolamine, and Europe and China respectively produced 511,000 tons and 396,000 tons. At the same year, North America's consumption accounted for about 28.85% of global consumption. Europe and China respectively consumed 394,000 tons and 549,000 tons of monoethanolamine. Global consumption has also increased from 1.811 million tons in 2012 to 1.919 million tons in 2017, with an annual growth rate of 1.17%. The global monoethanolamine market was valued at USD 2.46 billion in 2017.

At present, monoethanolamine in industries can be produced by the reaction of ammonia and ethylene oxide, and the reaction also produces diethanolamine and triethanolamine. In nature, all bacteria and eukaryotic cell comprises membrane lipids that comprises phosphatidylethanolamine. When phosphodiesterase hydrolyses the phosphatidylethanolamine, natural monoethanolamine is produced. But so far, biosynthesis of natural monoethanolamine in large-scales still cannot be realized in industries.

### SUMMARY OF THE DISCLOSURE

An object of the present disclosure is to provide a method for biosynthesizing monoethanolamine.

Another object of the present disclosure is to provide *Torulaspora delbrueckii* OMK-71 used in the above method.

A technical solution of the present disclosure is as follows.

A method for biosynthesizing monoethanolamine, which comprises the following steps:
(1) transferring activated *Torulaspora delbrueckii* OMK-71 into a fermentation medium containing a carbon source used to be metabolized by yeasts to promote growth and/or to produce monoethanolamine and culturing to obtain a fermentation broth; the *Torulaspora delbrueckii* OMK-71 has been deposited in the China Center for Type Culture Collection (Wuhan University, Wuhan, China) on December 27, 2019, with a deposit number of CCTCC NO: M20191124;
(2) adding the fermentation broth to a reaction buffer containing serine to perform a reaction to obtain a reaction solution; and
(3) purifying of the reaction solution obtained in step (2) to obtain monoethanolamine.

In a preferred embodiment, the carbon source comprises sucrose, fructose, xylose, ethanol, methanol, glycerol, glucose, cellulose, starch, or cellobiose. The carbon source can be provided to the yeasts during the entire cultivation period, or the yeasts can grow for a period of time in the presence of another energy such as protein, and the carbon source is only periodically provided in fed-batch culture.

In a preferred embodiment, the carbon source is glycerol.

In a preferred embodiment, a formulation of the reaction buffer containing serine comprises 18-22 g/L of glucose, 1-150 g/L of serine and 90-110 mg/L of Tween 80, and a potassium phosphate buffer is used to adjust pH to 7.1-7.3 to enable a final concentration of potassium phosphate to be 90-110 mM.

In a preferred embodiment, the formulation of the reaction buffer containing serine comprises 20g/L of glucose, 145 g/L of serine and 100 mg/L of Tween 80, and 1M potassium phosphate buffer is used to adjust the pH to 7.2 to enable the final concentration of potassium phosphate to be 100 mM.

In a preferred embodiment, a formulation of the fermentation medium comprises: 75-85 mL of 80-90% phosphoric acid, 2-4 g of CaSO₄, 50-60g of K₂SO₄, 10-15 g of KOH, 40-50 g of MgSO₄.7H₂O, 110-130 g of glycerol, 10-20 g of yeast extract, 10-20 g of peptone and 2-4 mL of antifoaming oil, and diluting to 3L with purified water.

In a preferred embodiment, the formulation of the fermentation medium comprises 80.1 mL of 85% phosphoric acid, 2.79 g of CaSO₄, 54.6 g of K₂SO₄, 12.39 g of KOH, and 44.7 g of MgSO₄.7H₂O, 120 g of glycerol, 15 g of yeast extract, 15 g of peptone and 3mL of antifoaming oil, and diluting to 3L with the purified water.

In a preferred embodiment, the purifying in step (3) comprises polyacrylamide flocculation, active carbon decolorization, ultrafiltration, adsorption and elution of cation exchange resin, and reduced pressure concentration in vacuum in sequence.

Another technical solution of the present disclosure is as follows.

*Torulaspora delbrueckii* OMK-71, characterized in that: which has been deposited at China Center for Type Culture Collection (Wuhan University, Wuhan, China) on December 27, 2019, with a deposit number of CCTCC NO: M 20191124.

A method for biosynthesizing monoethanolamine using *Torulaspora delbrueckii* OMK-71.

Compared with the existing techniques, the technical solution has the following advantages.
1. *Torulaspora delbrueckii* OMK-71 of the present disclosure can convert serine to biosynthesize monoethanolamine, and a monoethanolamine yield can reach 79 g/L or higher.
2. The present disclosure developes a method for biosynthesizing monoethanolamine from serine by *Torulaspora delbrueckii* OMK-71. The process is simple and is different from other monoethanolamine production methods, and it can be produced in large scales.

### BRIEF DESCRIPTION OF THE DRAWINGS

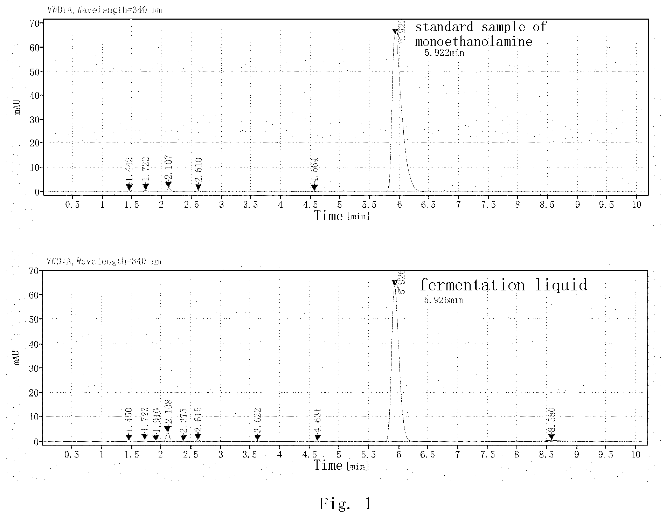

FIG. 1 is a high-performance liquid chromatogram of monoethanolamine of Embodiment 1 of the present disclosure, which proves that the obtained product is monoethanolamine.

### DETAILED DESCRIPTION OF THE EMBODIMENTS OF THE PRESENT DISCLOSURE

The technical solution of the present disclosure will be further described below in combination with the accompanying drawings and embodiments.

The present disclosure is based on the following discovery that all of bacteria and eukaryotic cells comprises membrane lipids that comprises phosphatidylethanolamine. When the phosphatidylethanolamine is hydrolysed by phosphodiesterase, natural monoethanolamine is produced. However, as monoethanolamine can inhibit growths of bacteria and eukaryotic cells to a certain extent, it is commonly difficult for bacteria and eukaryotic cells to produce high concentrations of monoethanolamine. Even in case it is produced, it is immediately used for glycerophospholipid metabolism.

In the present disclosure, the yeasts were isolated from marine mud beds in different regions, and 18S ribosomal RNA, 26S ribosomal RNA and internal transcription spacer (ITS) of each of the yeasts was amplified and sequenced by high-fidelity polymerase chain reaction (PCR) technology. The target sequences obtained were queried in a nucleotide sequence database, and the target sequences were used as reference sequences to perform BLAST, reciprocal BLAST or PSI-BLAST analysis in a non-redundant database. The yeasts were bred by a combined mutagenesis, preferably, the combined mutagenesis of the yeasts using ultraviolet rays and lithium chloride to select a yeast with a high monoethanolamine yield. The yeast (the resulting yeast) was used to biosynthesize natural monoethanolamine in large scales. The method comprises the following steps: providing the yeast used to prepare monoethanolamine in a presence of a carbon source; culturing the yeast in the presence of the carbon source; and purifying monoethanolamine from the yeast or from a culture supernatant of the yeast.

### Embodiment 1 Strain screening

### (1) Preparation of a cell suspension

A strain liquid of *Torulaspora delbrueckii* that grew at 28 °C and 250 rpm for 48 hours in a 50 mL flat-bottomed Erlenmeyer flask with 10 mL yeast extract peptone glucose (YPD) media was collected, and strains in the strain liquid were collected by centrifugation, and the strains were washed for 3 times with 10 mL purified water, then resuspended with 10 mL purified water, and placed in a 50 mL flat-bottomed Erlenmeyer flask. Five microliters of Tween 80 were added by a pipette, and the flask was shaken at 28°C and 250 rpm for 30 minutes to disperse the strains. A cell density of the yeasts was obtained by analyzing OD₆₀₀, and the cell density was adjusted to about 1 million cells/mL by adding an appropriate amount of purified water.

### (2) Ultraviolet ray mutagenesis

10 mL of the yeast suspension with the cell density of about 1 million cells/mL was taken and placed in a sterilized petri dish with a diameter of 90 mm. The petri dish was placed on a plate of a magnetic stirrer, with the lid of the petri dish removed. Ultraviolet irradiation was carried out at a position that was 30 cm vertically below an ultraviolet lamp, and the irradiation time was 20 min.

### (3) Lithium chloride mutagenesis

10 mL the yeast suspension after UV mutagenesis was taken, completely spread on YPD agar media containing 0.02% lithium chloride as a mutagen, wrapped with kraft paper, and placed in a 28 °C incubator for 72 hours.

### (4) Screening of the yeasts

Mutant strains that grew well and had relatively large single colony diameters were picked and streaked on YPD agar media, and cultured in a 28 °C constant temperature incubator for 48 hours. About 500 yeast mutant strains were picked, the mutant strains were inoculated in 50 mL flat-bottomed Erlenmeyer flasks with the 10 mL YPD media and grew at 28 °C and 250 rpm for 48 hours to obtain a stain solution. The strains in the strain solution were collected by centrifugation, and the strains were resuspended in a reaction buffer containing 1 g/L of serine. Samples were taken at 1 hour and 24 hours and pre-column derivatization were performed, and the content of monoethanolamine were analyzed by high performance liquid chromatography (HPLC). A yeast before a combined mutagenesis was used as a blank control.

A formulation of the YPD media was as follows: yeast extract 10 g/L, peptone 10 g/L and glucose 20 g/L.

A formulation of the YPD agar media was as follows: yeast extract 10 g/L, peptone 10 g/L, glucose 20 g/L and agar powder 15 g/L.

A formulation of the YPD agar media containing the 0.02% lithium chloride mutagen was as follows: yeast extract 10 g/L, peptone10 g/L, glucose 20 g/L, agar powder 15 g/L and lithium chloride 0.2 g/L.

A formulation of the reaction buffer containing 1g/L serine was as follows: glucose 20 g/L, serine 1 g/L and Tween 80 (100 mg/L). pH was adjusted to 7.2 by 1 molar (1 M) of potassium phosphate buffer to enable a final concentration of the potassium phosphate to be 100 mM.

After a combined mutagenesis by the ultraviolet rays and the lithium chloride, *Torulaspora delbrueckii* that can accumulate a certain amount of monoethanolamine in these strains was selected and named as PP1. Referring to Fig. 1, a monoethanolamine yield was about 2.4 mg/L. The mutant *Torulaspora delbrueckii* PP1 was stored at -80 °C with glycerol, and the monoethanolamine yield was stable after it was passaged 5 times.

### Embodiment 2 Re-screening of Torulaspora delbrueckii PP1

### (1) Preparation of a cell suspension

A strain liquid of *Torulaspora delbrueckii* PP1 that grew at 28 °C and 250 rpm for 48 hours in a 50 mL flat-bottomed Erlenmeyer flask with 10 mL YPD media was collected, and strains in the strain liquid were collected by centrifugation, and the strains were washed for 3 times with 10 mL purified water, then resuspended with 10 mL purified water, and placed in a 50 mL flat-bottomed Erlenmeyer flask. Five microliters of Tween 80 were added by a pipette and the flask was shaken at 28 °C and 250 rpm for 30 minutes to disperse the strains. A cell density of the *Torulaspora delbrueckii* PP1 was obtained by analyzing OD₆₀₀, and the cell density was adjusted to about 1 million cells/mL due to adding an appropriate amount of purified water.

### (2) Ultraviolet ray mutagenesis

10 mL of *Torulaspora delbrueckii* PP1 suspension with the cell density of about 1 million cells/mL was taken and placed in a sterilized petri dish with a diameter of 90 mm. The petri dish was placed on a plate of a magnetic stirrer, with the lid of the petri dish removed. Ultraviolet irradiation was carried out at a position that was 30 cm vertically below an ultraviolet lamp, and the irradiation time was 20 min.

### (3) Lithium chloride mutagenesis

10 mL of the *Torulaspora delbrueckii* PP1 suspension after UV mutagenesis was taken, completely spread on YPD agar media containing 0.02% lithium chloride mutagen, wrapped with kraft paper, and placed in a 28°C incubator for 72 hours.

### (4) Screening of Torulaspora delbrueckii PP1

500 mutant strains that grew well and had relatively large single colony diameters were picked and streaked on YPD agar media, and cultured in a 28 °C constant temperature incubator for 48 hours. The mutant strains were inoculated in 50 mL flat-bottomed Erlenmeyer flasks with the 10 mL YPD media and grew at 28 °C and 250 rpm for 48 hours to obtain a stain solution. The strains in the strain solution were collected by centrifugation, and the strains were resuspended in a reaction buffer containing 1g/L of serine. Samples were taken at 1 hour and 24 hours and pre-column derivatization were performed, and a monoethanolamine content was analyzed by HPLC. The *Torulaspora delbrueckii* PP1 before the re-screening was used as a control.

A formulation of the YPD media was as follows: yeast extract 10 g/L, peptone 10 g/L and glucose 20 g/L.

A formulation of the YPD agar media was as follows: yeast extract 10 g/L, peptone 10 g/L, glucose 20 g/L and agar powder 15 g/L.

A formulation of the YPD agar media containing the 0.02% lithium chloride mutagen was as follows: yeast extract 10 g/L, peptone 10 g/L, glucose 20 g/L, agar powder 15 g/L and lithium chloride 0.2 g/L.

A formulation of the reaction buffer containing 1g/L serine was as follows: glucose 20 g/L, serine 1 g/L and Tween 80 (100 mg/L). pH was adjusted to 7.2 by 1 molar (1M) of potassium phosphate buffer to enable a final concentration of the potassium phosphate to be 100 mM

After a combined mutagenesis by the ultraviolet rays and the lithium chloride, *Torulaspora delbrueckii* that can accumulate a certain amount of monoethanolamine in the strain was re-screened and named as PP2, which is used to output about 30.1 mg/L of monoethanolamine. The mutant *Torulaspora delbrueckii* PP2 was stored at -80 °C with glycerol, and the monoethanolamine yield was stable after it was passaged 5 times.

The *Torulaspora delbrueckii* PP2 was processed by the combined mutagenesis by the ultraviolet rays and the lithium chloride again, and *Torulaspora delbrueckii* with a high monoethanolamine yield was finally selected and named *Torulaspora delbrueckii* OMK-71 after multiple rounds of the combined mutagenesis. It can produce about 20.2 g/L monoethanolamine by a shake-flask fermentation in the flat-bottomed Erlenmeyer flask. *Torulaspora delbrueckii* OMK-71 obtained by the mutagenesis was stored at -80 °C with glycerol, and the monethanolamine yield was stable after it was passaged 5 times.

### Embodiment 3: Fermentation production of natural monoethanolamine

*Torulaspora delbrueckii* OMK-71 obtained in Embodiment 2 was inoculated into a 250 mL flat-bottomed Erlenmeyer flask containing 50 mL YPD media and grew at 28 °C and 250 rpm for 48 hours to activate the strains to obtain a first-stage seed solution. The first-stage seed solution was transferred to a 1 L flat-bottomed Erlenmeyer flask containing 150 mL YPD media with an inoculum dose of 1% and grew at 28 °C and 250 rpm for 18 hours to obtain a second-stage seed liquid. 100 mL of the second-stage seed liquid was inoculated into 3 L of a fermentation medium. A fermentation was possessed in a 5 L small-test bioreactor, and its temperature and pH were respectively controlled at 28-30 °C and 5.0-6.0. During the whole operation, the air flow speed was maintained at 3 L/min, the pressure was maintained at 0.08 Mpa, the pH was controlled by adding ammonia water, and the initial stirring speed was 500 rpm.

After 24 hours of the fermentation, the dissolved oxygen concentration (DO) descended to 50%, glycerol was then supplemented, and the stirring speed was increased to 600 rpm until the detected biomass reached 200 mg/mL (wet weight). After 120 hours, the strain cell density of the fermentation liquid reached saturation, and a maximum OD₆₀₀ value can reach 400.

The fermentation liquid was diluted into a 30 L bioreactor with a reaction buffer containing 145 g/L serine, and the temperature, the pH and the stirring speed were respectively controlled at 30 °C, 7.2 and 200 rpm. During the whole operation, the air flow speed was maintained at 3 L/min, and the pressure was maintained at 0.08 Mpa. After it was reacted for 7 hours, a reaction solution was obtained, in which the concentration of monoethanolamine was 79 g/L.

A formulation of the YPD medium was as follows: yeast extract 10 g/L, peptone 10 g/L and glucose 20 g/L.

A formulation of the fermentation medium was as follows: 85% phosphoric acid 80.1 mL, CaSO₄ 2.79 g, K₂SO₄ 54.6 g, KOH 12.39 g, MgSO₄.7H₂O 44.7 g, glycerol 120 g, yeast extract 15 g, peptone 15 g and defoaming oil 3 mL, and it was set to a 3L volume by purified water.

A formulation of the reaction buffer containing 145g/L serine was as follows: glucose 20 g/L, serine 145 g/L and Tween 80 (100 mg/L), and pH was adjusted to 7.2 by 1 molar (1M) of potassium phosphate buffer to enable a final concentration of the potassium phosphate to be 100 mM, and the rest is water.

### Embodiment 4 Extraction of natural monoethanolamine

(1) Flocculation: 50 L of the reaction solution obtained in Embodiment 3 was taken, the pH of the reaction solution was adjusted to 4.0 by hydrochloric acid, the temperature of the solution was 30 °C, and cationic polyacrylamide was added to enable its concentration to be 10 mg/L. The reaction solution was stirred for 90 minutes, left to stand for 6 hours, and then centrifuged to remove precipitate to obtain a clear liquid.
(2) Decolorization: powdered active carbon was added to the clear liquid, the addition dose was 5 g/L, the temperature was increased to 65°C, and the clear liquid was stirred and decolorized for 60 minutes, and the active carbon was removed to obtain a decolorization liquid by filtration.
(3) Ultrafiltration: the decolorization liquid was Ultra-filtered with an ultrafilter membrane, and a molecular weight cut-off was 2000 Da. When the decolorization liquid to be cut off to remain 5-8 L, 5-10 L purified water was added for washing, the washing was performed for 3 times, and an ultrafiltrate was obtained after mixing.
(4) Adsorption and elution: the pretreated 732 cation exchange resin was filled into a Φ150X 2000 mm column, and the ultrafiltrate was loaded on the column at a flow rate of 400 mL/min. After the ultrafiltrate was completely loaded on the column, the column was washed with 70 L of water of pH 4.0. After it was washed, the elution was performed with 0.2 mol/L ammonia water at the same flow rate to obtain an eluate comprising a high proportion of the monoethanolamine.
(5) Concentration: the eluate was concentrated in vacuum at a temperature no more than 65 °C to obtain a crude product of the monoethanolamine with a purity of 98%.

The invention may be summarized as follows: The present disclosure discloses a method for biosynthesizing monoethanolamine, which comprises the following steps: (1) transferring activated Torulaspora delbrueckii OMK-71 into a fermentation medium containing a carbon source used to be metabolized by yeasts to promote growth and/or to produce monoethanolamine and culturing to obtain a fermentation broth; (2) adding the fermentation broth to a reaction buffer containing serine to perform a reaction to obtain a reaction solution; and (3) purifying of the reaction solution obtained in step (2) to obtain monoethanolamine. Torulaspora delbrueckii OMK-71 of the present disclosure can be used to biosynthesize monoethanolamine in large scales, and a monoethanolamine yield can reach 79 g/L or higher.

The aforementioned embodiments are merely some embodiments of the present disclosure, and the scope of the disclosure is not limited thereto. Thus, it is intended that the present disclosure cover any modifications and variations of the presently presented embodiments provided they are made without departing from the appended claims and the specification of the present disclosure.

## Claims

1. A method for biosynthesizing monoethanolamine, **characterized in that**, comprising the following steps:
(1) transferring activated *Torulaspora delbrueckii* OMK-71 into a fermentation medium containing a carbon source used to be metabolized by yeasts to promote growth and/or to produce monoethanolamine and culturing to obtain a fermentation broth; the *Torulaspora delbrueckii* OMK-71 has been deposited in the China Center for Type Culture Collection on December 27, 2019, with a deposit number of CCTCC NO: M20191124;
(2) adding the fermentation broth to a reaction buffer containing serine to perform a reaction to obtain a reaction solution; and
(3) purifying of the reaction solution obtained in step (2) to obtain monoethanolamine.

2. The method according to claim 1, **characterized in that**: the carbon source comprises sucrose, fructose, xylose, ethanol, methanol, glycerol, glucose, cellulose, starch, or cellobiose.

3. The method according to claim 2, **characterized in that**: the carbon source is glycerol.

4. The method according to any one or more of claims 1 to 3, a formulation of the reaction buffer containing serine comprises 18-22 g/L of glucose, 1-150 g/L of serine and 90-110 mg/L of Tween 80, and adjusting pH to 7.1-7.3 with potassium phosphate buffer to enable a final concentration of potassium phosphate to be 90-110 mM.

5. The method according to claim 4, **characterized in that**: the formulation of the reaction buffer containing serine comprises 20 g/L of glucose, 145 g/L of serine and 100 mg/L of Tween 80, and adjusting the pH to 7.2 by 1M of potassium phosphate buffer to enable the final concentration of potassium phosphate to be 100 mM

6. The method according to any one or more of claims 1 to 5, **characterized in that**: a formulation of the fermentation medium comprises 75-85 mL of 80-90% phosphoric acid, 2-4 g of CaSO₄, 50-60 g of K₂SO₄, 10-15 g of KOH, 40-50 g of MgSO₄.7H₂O, 110-130 g of glycerol, 10-20 g of yeast extract, 10-20 g of peptone and 2-4 mL of antifoaming oil, and diluting to 3L with purified water.

7. The method according to claim 6, **characterized in that**: the formulation of the fermentation medium comprises 80.1 mL of 85% phosphoric acid, 2.79 g of CaSO₄, 54.6 g of K₂SO₄, 12.39 g of KOH, 44.7 g of MgSO₄.7H₂O, 120 g of glycerol, 15 g of yeast extract, 15 g of peptone and 3 mL of antifoaming oil, and diluting to 3L with the purified water.

8. The method according to any one or more of claims 1 to 7, **characterized in that**: the purifying in step (3) comprises polyacrylamide flocculation, active carbon decolorization, ultrafiltration, adsorption and elution of cation exchange resin, and reduced pressure concentration in vacuum in sequence.

9. *Torulaspora delbrueckii* OMK-71, **characterized in that**: which has been deposited at China Center for Type Culture Collection on December 27, 2019, with a deposit number of CCTCC NO: M 20191124.

10. A method for biosynthesizing monoethanolamine using *Torulaspora delbrueckii* OMK-71 according to claim 9.
